# EUROPEAN PATENT APPLICATION

(11) **EP 2 842 489 A1**
(43) Date of publication of application: **04.03.2015**
(21) Application number: 14173026.7
(22) Date of filing: 18.06.2014
(51) Int. Cl.: A61B 5/117, A61B 5/00, G06F 21/32, G06K 9/00

(54) **Biometric authentication apparatus**

(30) Priority: 30.08.2013 JP 2013178803
(71) Applicant: Hitachi-Omron Terminal Solutions, Corp., Shinagawa-ku Tokyo 141-8576 (JP)
(72) Inventor: Yamamoto, Naoto, Tokyo, 141-8576 (JP); Imaizumi, Atsuhiro, Tokyo, 141-8576 (JP)
(74) Representative: MERH-IP Matias Erny Reichl Hoffmann

(57) **Abstract**

A biometric authentication apparatus includes: a living body disposing portion (105, 106) on which a living body (301, 1101) of a user is disposed; a light source portion (103) for irradiating light to the living body (301, 1101) disposed on the living body disposing portion (105, 106); two or more imaging portions (101, 102) for imaging transmitted light which light irradiated from the light source portions (103) is transmitted through the living body (301,1101) to become; and a control portion (110) for controlling the living body disposing portion (105, 106), the light source portion (103), and the two or more imaging portions (101, 102). In this case, the imaging portions (101, 102) capture a first biometric image (501) used in biometric authentication, and a second biometric image (502) used to calculate a correction coefficient based on which the first biometric image (501) is corrected. The control portion (110) calculates the correction coefficient, and corrects the first biometric image (501) by using the correction coefficient.

## Description

### BACKGROUND OF THE INVENTION

### Field of the Invention

The present invention relates to a biometric authentication apparatus.

### Description of the Related Art

A biometric authentication apparatus for authenticating a user by using biometric information is utilized in various instruments and systems. When the person himself/herself is authenticated by using the biometric authentication apparatus, the user carries out an action for holding up a predetermined portion of his/her body such as a finger or an eye(s) over the biometric authentication apparatus, or an action for peeking at the biometric authentication apparatus (operation for user authentication), thereby reading the biometric information on the user. Also, a feature amount extracted from the biometric information is compared with a feature amount of biometric information which the person himself/herself previously registered to calculate the degree of coincidence in the comparison. Also, the judgment for the person himself/herself is carried out depending on the judgment as to whether or not the degree of coincidence in the comparison exceeds a predetermined threshold value.

When the personal authentication is carried out with the biometric authentication apparatus, as one of primary factors causing the personal false rejection in which the rejection is carried out by mistake in spite of the registrant himself/herself, there is given the case where the disposition in a phase of the authentication is different from that of the living body in the phase of the registration. The background art in this technical field includes a technique disclosed in JP-A-2009-178300. This official gazette gives a description as follows: A finger disposed on a disposition board is imaged with a finger position detecting camera, thereby detecting the finger's position and judging whether or not the finger's position is right on the image information. As a result, if the finger's position is judged to be false, then, a right finger position image is displayed on a display portion to teach a user the correct position of his/her finger on the disposition board.

### SUMMARY OF THE INVENTION

When the technique described in JP-A-2009-178300 is used, it is also possible to acquire the biometric information in the right position. However, when the user keeps his/her living body disposing in the shape which is unfavorable for the authentication of the living body's shape because of the apparatus which instructs the user to reset his/her living body, the biometric information cannot be properly acquired, which results in that the authentication becomes difficult to carry out in some cases. For example, the disposition style, which causes the living body to be distorted, such as the case where the living body is inclined, or the case where the living body is bent at a joint becomes difficult to authenticate because the biometric information is also distorted.

In the light of the foregoing, it is therefore an object of the present invention to provide a biometric authentication apparatus which is capable of acquiring biometric information even in the case where a living body is not properly disposed when a user disposes his/her living body, thereby carrying out authentication of the user.

In order to solve the problems described above, according to an embodiment of the present invention, there is provided a biometric authentication apparatus including: a living body disposing portion in which a living body of a user is disposed; a light source portion for irradiating light to the living body disposed on the living body disposing portion; two or more imaging portions for imaging transmitted light which the light irradiated from the light source portion is transmitted through the living body to become; and/or a control portion for controlling the living body disposing portion, the light source portion, and the two or more imaging portions. In this case, the two or more imaging portions capture a first biometric image used in biometric authentication, and a second biometric image based on which a correction coefficient used to correct the first biometric image is calculated. Also, the control portion calculates the correction coefficient and corrects the first biometric image by using the correction coefficient.

As set forth hereinabove, according to the present invention, when the detection of the living body is carried out with the imaging device mounted to the biometric authentication apparatus, so that the state of the living body, such as the inclination of the living body, the bend of the living body at the joint, or the warp of the living body has the shape which is unfavorable for the authentication, the biometric information is corrected depending on the state of the living body to enable the living body to be authenticated, thereby enhancing the convenience of the biometric authentication apparatus.

Other objects, features and advantages of the invention will become apparent from the following description of the embodiments of the invention taken in conjunction with the accompanying drawings.

### BRIEF DESCRIPTION OF THE DRAWINGS

FIG. 1 is a view showing an external appearance of a biometric authentication apparatus according to a first embodiment of the present invention;
FIG. 2 is a block diagram showing a configuration of the biometric authentication apparatus shown in FIG. 1;
FIG. 3A is a cross-sectional view showing disposition of constituent parts or components of the biometric authentication apparatus shown in FIG. 1;
FIG. 3B is a top plan view showing the disposition of the constituent parts or components of the biometric authentication apparatus shown in FIG. 1;
FIG. 4 is a flow chart explaining registration processing for registering biometric information with the biometric authentication apparatus shown in FIG. 1;
FIG. 5 is a flow char explaining authentication processing for authenticating the biometric information with the biometric authentication apparatus shown in FIG. 1;
FIG. 6 is a view explaining biometric images captured by imaging portions, respectively, with the biometric authentication apparatus shown in FIG. 1;
FIG. 7 is a view explaining detection of a contour of the biometric image with the biometric authentication apparatus shown in FIG. 1;
FIGS. 8A to 8C are respectively views explaining detection of an inclination and a bend of the biometric information from the biometric images with the biometric authentication apparatus shown in FIG. 1;
FIGS. 9A to 9C are respectively views explaining image correction of the biometric image with the biometric authentication apparatus shown in FIG. 1;
FIG. 10 is a flow chart explaining authentication processing with a biometric authentication apparatus according to a second embodiment of the present invention;
FIG. 11 is a view showing disposition of constituent parts or components in a biometric authentication apparatus according to a third embodiment of the present invention;
FIG. 12 is a flow chart explaining authentication processing with the biometric authentication apparatus according to the third embodiment of the present invention;
FIG. 13 is a view showing disposition of constituent parts or components in a biometric authentication apparatus according to Modified Change 1 of the first, second and third embodiments of the present invention; and
FIG. 14 is a view showing an external appearance of a biometric authentication apparatus according to Modified Change 2 of the first, second and third embodiments of the present invention.

### DETAILED DESCRIPTION OF THE EMBODIMENTS

Hereinafter, embodiments of the present invention will be described in detail with the accompanying drawings.

### FIRST EMBODIMENT

### A. Configuration of Apparatus

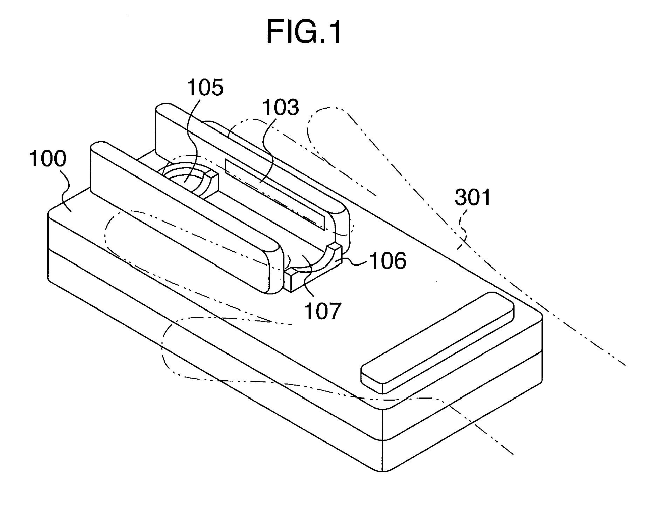

A first embodiment of the present invention will be described below with reference to FIGS. 1 to 9. First of all, a configuration of a biometric authentication apparatus 100 according to the first embodiment of the present invention will now be described. FIG. 1 is an external view of the biometric authentication apparatus 100 of the first embodiment. As shown in FIG. 1, the biometric authentication apparatus 100 includes light source portions 103, a fingertip disposing portion 105, a finger root disposing portion 106, and an opening portion 107.

The fingertip disposing portion 105 is a support portion which supports a fingertip of a living body 301 of a user. Also, the finger root disposing portion 106 is a support portion which supports the root of the finger of the living body 301 of the user. The light source portions 103 include infrared LEDs or the like which emit near infrared light, and respectively irradiate the infrared light from right- and left-hand sides to the finger of the user which is disposed (supported) on the fingertip disposing portion 105 and the finger root disposing portion 106. The transmitted light of the near infrared light irradiated from the light source portions 103 is guided to a first imaging portion 101 and a second imaging portion 102 which will be described later through the opening portion 107. It is noted that the opening portion 107 may include a filter which cuts light from the outside, a panel, made of acryl, which prevents dust from entering the biometric authentication apparatus 100, or the like.

That is to say, the living body 301 (finger) of the user is disposed on the fingertip disposing portion 105 and the finger root disposing portion 106, whereby the infrared light is irradiated to both sides of the living body 301 thus disposed by the light source portions 103. Then, the light transmitted through the living body 301 passes through the opening portion 107 to be imaged by the first imaging portion 101 and the second imaging portion 102 which are located below the opening portion 107, thereby imaging (acquiring) biometric information (biometric image) of the user.

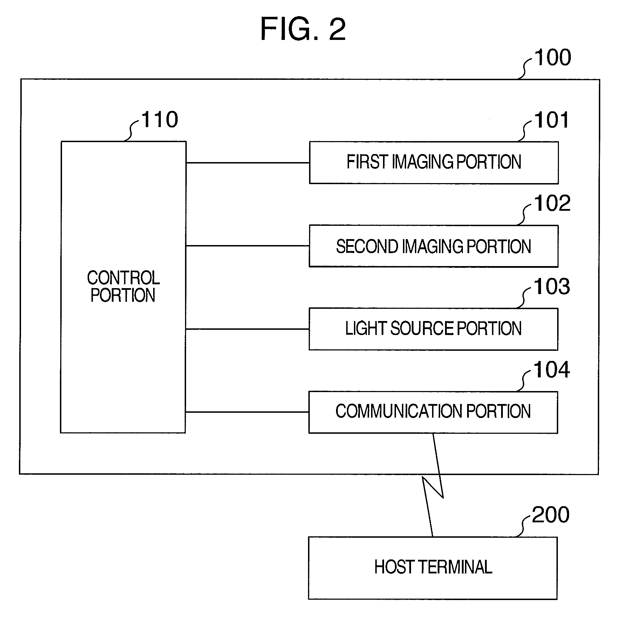

FIG. 2 is a functional block diagram representing functional blocks of the biometric authentication apparatus 100 of the first embodiment. In the biometric authentication apparatus 100, a control portion 110 controls the first imaging portion 101, the second imaging portion 102, the light source portions 103, and a communication portion 104. In addition, the biometric authentication apparatus 100 is connected to a host terminal 200 through the communication portion 104. If the biometric authentication apparatus 100 of the first embodiment, for example, is incorporated in an Automated Teller Machine (ATM) installed in a financial institution, a convenience store or the like, the ATM corresponds to the host terminal 200. In addition, when the biometric authentication apparatus 100, for example, is a terminal installed in a window of the financial institution, a terminal for a clerk in charge which the clerk in charge uses corresponds to the host terminal 200.

Each of the first imaging portion 101 and the second imaging portion 102 is an imaging portion which images transmitted light guided thereto through the opening portion 107. Each of the light source portions 103 includes an infrared LED or the like which can irradiate near infrared light. In this case, the control portion 110 controls an irradiation timing and a quantity of light of the infrared LED. Therefore, the living body 301 can be imaged in consideration of an influence of light from the outside in accordance with the control for the quantity of light made by the control portion 110.

The communication portion 104 transmits biometric data (referred to as biometric information for registration as well) having a state which becomes suitable for the registration by executing processing in the control portion 110 described later for the biometric information obtained through the imaging by the first imaging portion 101 and the second imaging portion 102 to the host terminal 200. In this case, the processing executed in the control portion 110 will be described later. In addition, the communication portion 104 receives biometric data for authentication (referred to as biometric information for authentication as well) from the host terminal 200.

The control portion 110 is a control portion for controlling the first imaging portion 101, the second imaging portion 102, the light source portions 103, and the communication portion 104 which are all connected the control portion 110. In addition, the control portion 110 executes the registration processing and authentication processing for the biometric information on the user. For example, after the quantities of light from the light source portions 103 have been adjusted to a state which is suitable for the imaging of the living body 301 of the user, the control for the first imaging portion 101, the second imaging portion 102, the light source portions 103, and the communication portion 104 of the biometric authentication apparatus 100 is carried out. Also, the living body 301 of the user is imaged with the first imaging portion 101 and the second imaging portion 102. After that, a blood vessel pattern becoming feature information in a phase of authentication of the person himself/herself is extracted from the biometric image captured with the first imaging portion 101. Also, data on the blood vessel pattern thus extracted is outputted as registration data to the host terminal 200 through the communication portion 104. It is noted that on this occasion, a shape of the living body 301 is detected from the biometric image captured with the second imaging portion 102, and the output of the biometric data is stopped when the state of the living body 301 is judged to be unsuitable for the registration. This series of processing is referred to as registration processing as well. In addition, the blood vessel pattern may also be acquired from the image of the living body 301 captured with the second imaging portion 102.

In addition, the data on the blood vessel pattern thus extracted is compared with the registration data which was previously registered to calculate the degree of coincidence in the comparison. Also, the authentication of the person himself/herself is carried out depending on the judgment as to whether or not the degree of coincidence thus calculated exceeds a predetermined threshold value. Also, the authentication result is outputted to the host terminal 200 through the communication portion 104. This series of processing is referred to as authentication processing as well. It is noted that the control portion 110 includes a CPU, and a storage medium such as a memory, and develops a program to the memory, so that the control portion 110 executes image processing for the biometric image captured in processing by the CPU, and the like.

It should be noted that correction of the image of the living body 301 imaged with the first imaging portion 101 will be described later. In addition, a configuration may also be adopted in which a sensor for detecting a pressure is provided in each of the fingertip disposing portion 105 and the fingertip disposing portion 106, and the control portion 110 detects an output signal from the sensor, thereby controlling a timing of irradiation of the infrared light from the light source portions 103.

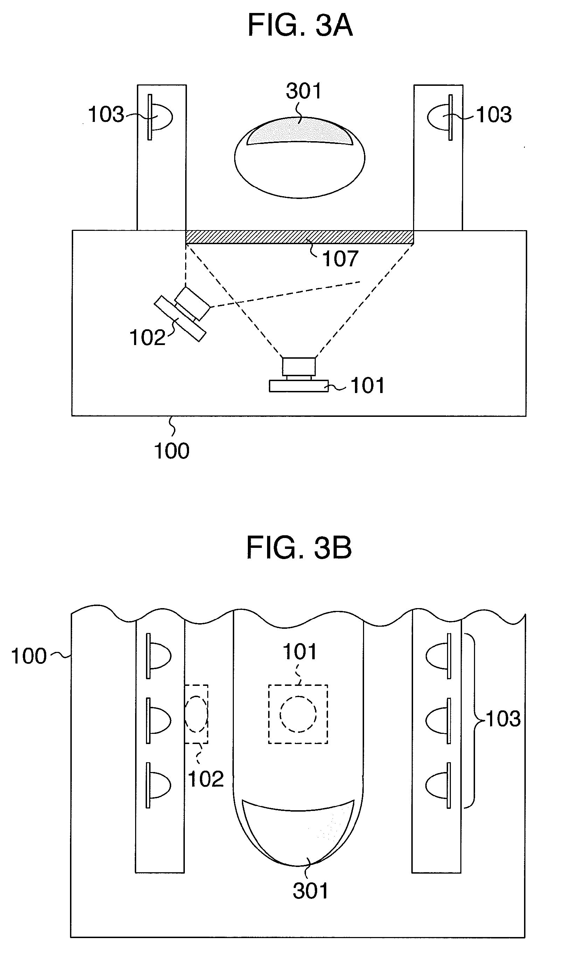

Next, a description will be given below with respect to the disposition of the constituent elements of the biometric authentication apparatus 100 of the first embodiment. FIG. 3A is a cross-sectional view showing the disposition of the constituent elements of the biometric authentication apparatus 100 of the first embodiment. In addition, FIG. 3B is a top plan view showing the disposition of the constituent elements of the biometric authentication apparatus 100 of the first embodiment. The light source portions 103 irradiate the near infrared light to the both sides of the living body 301 exhibiting the finger of the user. As shown in FIGS. 3A and 3B, the first imaging portion 101 is disposed in a position where the central front of the living body portion 301 can be imaged. Also, the second imaging portion 102 is disposed in a position where the side of the living body portion 301 can be imaged. It is noted that although in FIG. 3B, plural light source portions 103 are disposed on one side, one light source portion may be disposed, or one plate-like light source may also be disposed.

In other words, the first imaging portion 101 is located just under, a central portion at which a straight line perpendicularly intersecting with the light source portions 103, and an axis passing through the center of the biometric authentication apparatus 100 (or the opening portion 107) intersect with each other, with respect to the opening portion 107. Also, the second imaging portion 102 is located in the vicinity of the first imaging portion 101 (preferably, on the same axis as that of the first imaging portion 101 which is parallel with a short side of the biometric authentication apparatus 100 in a top plan view).

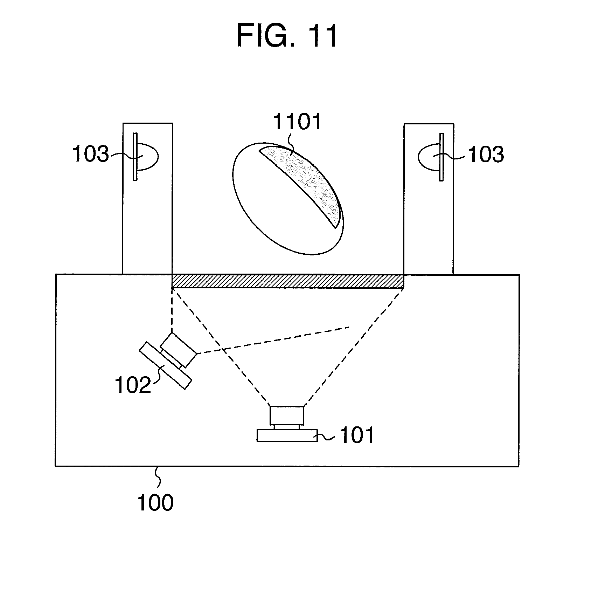

It is noted that an image captured by imaging the central front of the living body portion 301 is referred to as a first biometric image, and an image captured by imaging the side of the living body portion 301 is referred to as a second biometric image. In addition, the first biometric image is referred to as an image for biometric authentication as well, and the second biometric image is referred to as an image for correction as well. In addition, although in FIG. 3A, the first imaging portion 101 captures the first biometric image, as shown in FIG. 11, the second imaging portion 102 captures the first biometric image in some cases.

### B. Registration Processing for Biometric Information

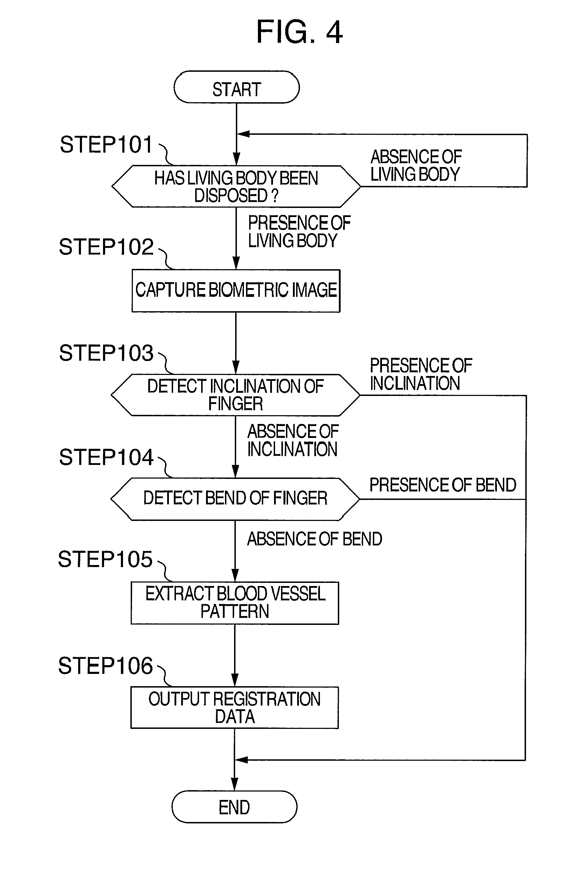

A description will be given below with respect to registration processing for the biometric information with the biometric authentication apparatus 100 of the first embodiment. FIG. 4 is a flow chart explaining the registration processing for the biometric information executed by using the biometric authentication apparatus 100 of the first embodiment.

In processing in Step 101, it is detected whether or not the living body 301 of the user has been disposed on the biometric authentication apparatus 100. While it is judged that the living body 301 of the user has not been disposed on the biometric authentication apparatus 100, the processing in Step 101 is repetitively executed (Step 101: absence of the living body). On the other hand, when it is judged that the living body 301 of the user has been disposed on the biometric authentication apparatus 100, the registration processing proceeds to processing in next Step 102 (Step 101: presence of the living body). In the processing in Step 102, after quantities of light emitted from the light source portions 103 mounted to the biometric authentication apparatus 100 have been adjusted to a state suitable for the imaging of the living body 301, the biometric images are captured by using the first imaging portion 101 and the second imaging portion 102, respectively.

In processing in Step 103, detection of an inclination of the living body 301 is carried out by using the second biometric image which was captured by imaging the side of the living body 301 with the second imaging portion 102. Details of the detection of the inclination of the living body 301 will be described later. When it is detected that the living body 301 is inclined to exceed a prescribed angle, the registration processing is stopped because an inclination state is judged to be unsuitable for the registration (Step 103: presence of the inclination). On the other hand, when it is detected that the living body 301 is not inclined to exceed the prescribed angle, the registration processing proceeds to processing in next Step 104 (Step 103: absence of the inclination).

Next, in processing in Step 104, detection of a bend of the living body 301 is carried out by using the second biometric image which was captured by imaging the side of the living body 301 with the second imaging portion 102. Details of the detection of the bend of the living body 301 will be described later. When it is detected that the living body 301 is bent at a joint to exceed a prescribed angle, the registration processing is stopped because a bend state is judged to be unsuitable for the registration (Step 104: presence of the bend). On the other hand, when it is detected that the living body 301 is not bent at the joint to exceed the prescribed angle, the registration processing proceeds to processing in next Step 105 (Step 104: absence of the bent).

In processing in Step 105, the blood vessel pattern becoming the feature information in the phase of authentication of the person himself/herself is extracted by using the first biometric image which was captured by imaging the front of the living body 301 with the first imaging portion 101. In processing in Step 106, the blood vessel pattern extracted in the processing in Step 105 is converted into data having a form which is suitable for comparison for the authentication of the person himself/herself. The resulting data is outputted as biometric registration data to the host terminal 200. The registration processing for the biometric information is completed through the six pieces of processing in Steps 101 to 106.

### C. Authentication Processing for Biometric Information

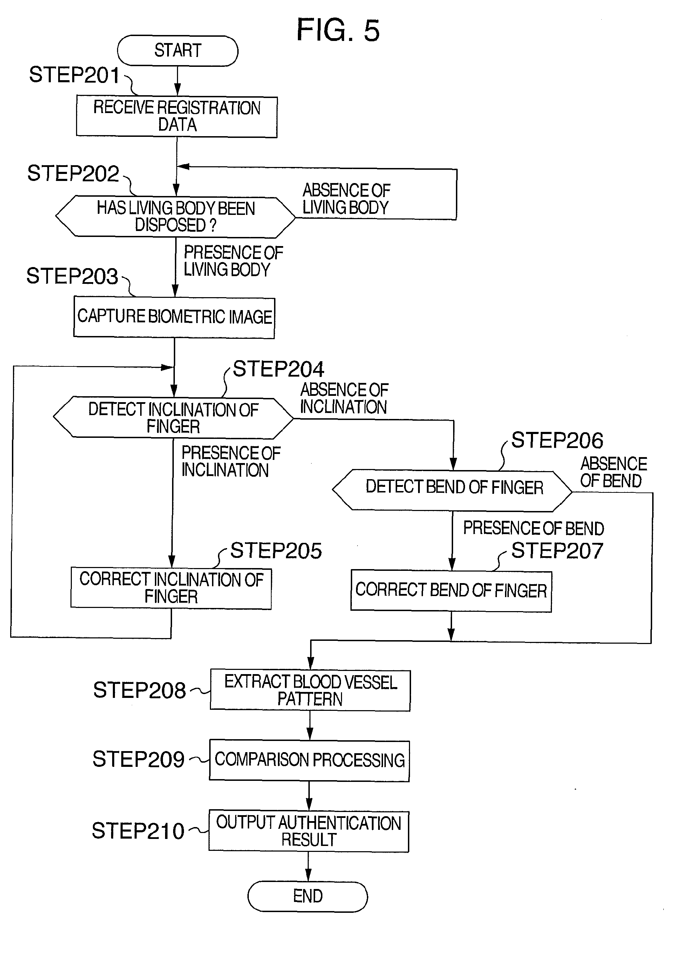

A description will be given below with respect to the biometric authentication processing in the biometric authentication apparatus 100 of the first embodiment. FIG. 5 is a flow chart explaining the biometric authentication processing using the biometric authentication apparatus 100 of the first embodiment.

In processing in Step 201, the biometric authentication apparatus 100 receives the biometric registration data which has been delivered thereto from the host terminal 200 through the communication portion 104. At this time, the biometric registration data which has been delivered from the host terminal 200 to the biometric authentication apparatus 100 is the biometric registration data which was previously registered in the registration processing for the biometric information described above. In processing in Step 202, it is detected whether or not the living body 301 has been disposed on the biometric authentication apparatus 100. While it is judged that the living body 301 has not been disposed on the biometric authentication apparatus 100, the processing in Step 202 is repetitively executed (Step 202: absence of the living body 301). On the other hand, when it is judged that the living body 301 has been disposed on the biometric authentication apparatus 100, the authentication processing proceeds to processing in next Step 203 (Step 202: presence of the living body 301).

In the processing in Step 203, after the quantities of light emitted from the light source portions 103 mounted to the biometric authentication apparatus 100 have been adjusted to the state suitable for the imaging of the living body 301, the biometric images are captured by using the first imaging portion 101 and the second imaging portion 102, respectively. In processing in Step 204, the detection of an inclination of the living body 301 is carried out by using the second biometric image which was captured by imaging the side of the living body 301 with the second imaging portion 102. When it is detected that the living body 301 is inclined to exceed a prescribed angle, the authentication processing proceeds to processing in Step 205 (Step 204:presence of the inclination). On the other hand, when it is judged that the living body 301 is not inclined to exceed the prescribed angle, the authentication processing proceeds to processing in Step 206 (Step 204: absence of the inclination).

In processing in Step 205, processing for correcting an inclination of the living body 301 is executed. When the living body 301 is disposed on the biometric authentication apparatus 100 to have an inclination, the first biometric image which was captured by imaging the central front of the living body 301 becomes an image which is distorted to have a trapezoidal shape depending on the inclination of the living body 301. The first biometric image is corrected in image processing in such a way that the state of the living body 301 becomes a state in which the living body 301 is disposed straight without being inclined, whereby it is possible to obtain the biometric image free from the inclination. After the correction for the inclination has been carried out, the judgment processing in Step 204 is executed again, and the authentication processing proceeds to processing in next Step 206.

In the processing in Step 206, detection of the bend of the living body 301 at the joint is carried out by using the second biometric image which was captured by imaging the side of the living body 301 with the second imaging portion 102. When it is detected that the living body 301 is bent at the joint to exceed a prescribed angle, the authentication processing proceeds to processing in Step 207 (Step 206: presence of the bend). On the other hand, when it is detected that the living body 301 is not bent at the joint to exceed the prescribed angle, the authentication processing proceeds to processing in Step 208 (Step 206: absence of the bend).

In the processing in Step 207, processing for correcting the bend of the living body 301 is executed. When the living body 301 is disposed on the biometric authentication apparatus 100 to be bent at the joint, the first biometric image which was captured by imaging the central front of the living body 301 becomes an image which is distorted in two directions to have trapezoidal shapes with the joint position of the living body 301 as a boundary. The first biometric image is corrected in the image processing in such a way that the state of the living body 301 becomes equal to a state in which the living body 301 is disposed straight without bending the living body 301 at the joint, whereby it is possible to obtain the biometric image free from the bend.

In the processing in Step 208, a blood vessel pattern for comparison is extracted by using the biometric image free from the inclination and the bend. In processing in Step 209, comparison processing for the blood vessel pattern is executed. The comparison based on pattern matching is carried out by using both of the biometric registration data previously registered, and the blood vessel pattern for comparison obtained in the processing in Step 208, thereby calculating the degree of coincidence in the pattern matching. In processing in Step 210, the judgment for authentication is carried out based on the degree of coincidence obtained in the processing in Step 209, and the judgment result is outputted to the host terminal 200. The authentication processing is completed through the ten pieces of processing in Steps 201 to 210.

It is noted that although the flow chart of FIG. 5 is set as the flow in which after the inclination of the finger has been detected (Step 204), the bend of the finger is detected, alternatively, a flow may also be adopted in which after the bend of the finger has been formerly detected to correct the bend of the finger, the inclination of the finger is detected. In addition, although in the flow chart of FIG. 5, after the inclination of the finger has been corrected, the detection of the finger is carried out again, alternatively, a flow may be adopted in which the authentication processing proceeds to the processing in Step for detecting the bend of the finger without detecting the inclination of the finger, or when the bend and inclination of the finger are not caused at the same time, the blood vessel pattern is extracted without proceeding to Step for detecting the bend of the finger. This also applies to flow charts in other embodiments which will be described later.

### D. Capturing of Biometric Image

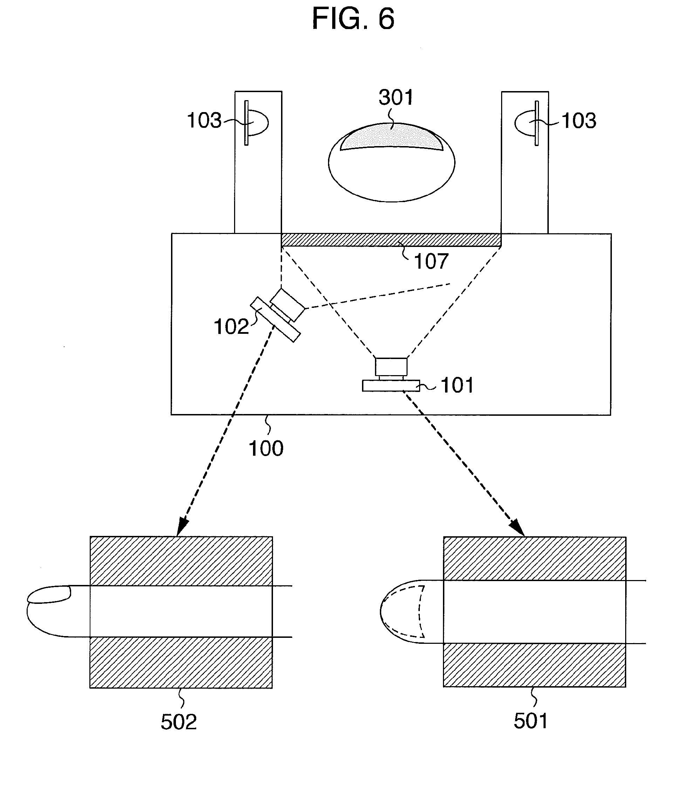

FIG. 6 is a view explaining the capturing of the biometric image either in the processing in Step 102 of FIG. 4 or in the processing in Step 203 of FIG. 5. After the quantities of light from the light source portions 103 mounted to the biometric authentication apparatus 100 have been adjusted to the state suitable for the imaging of the living body 301, the biometric images are captured by using the first imaging portion 101 and the second imaging portion 102, respectively. At this time, the first biometric image 501 which was captured by imaging the central front of the living body 301 is obtained with the first imaging portion 101. In addition, the second biometric image 502 which was captured by imaging the side of the living body 301 is obtained with the second imaging portion 102.

### E. Detection of Inclination and Bend of Living Body

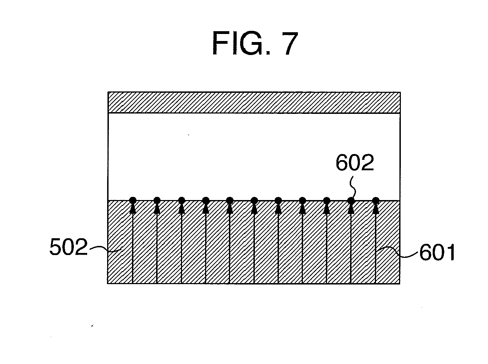

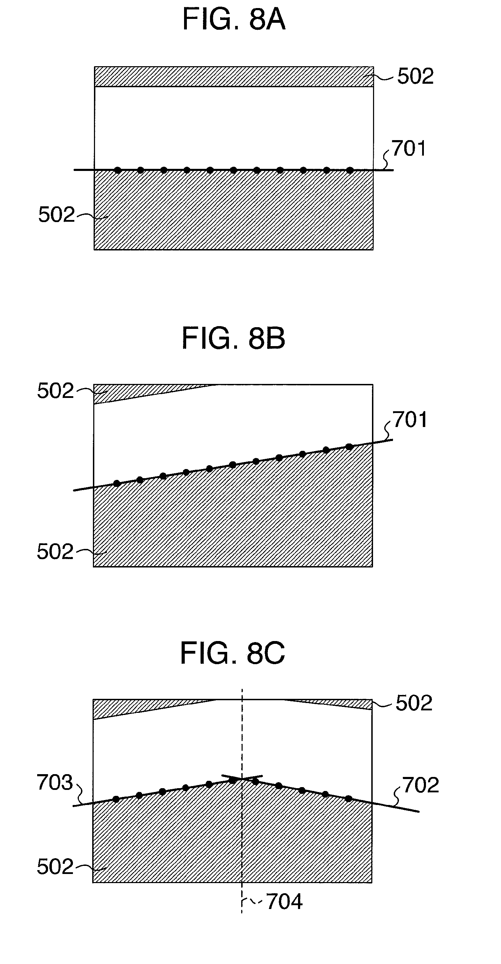

A method of detecting the inclination of the living body 301, and the bend of the living body 301 at the joint in the detection of the inclination of the living body 301 in the processing in Step 103, and the detection of the bend of the living body 301 in the processing in Step 104, or the detection of the inclination of the living body 301 in the processing in Step 204, and the detection of the bend of the living body 301 in the processing in Step 205 will be described below with reference to FIG. 7, and FIGS. 8A to 8C.

FIG. 7 is a view explaining processing for detecting a position of a contour of the imaged living body 301 from the second biometric image 502. In the processing for detecting a contour line of the living body 301, the second biometric image 502 is used which was captured by imaging the side of the living body 301. In the second biometric image 502, the inside of the living body 301 shows high luminance (white), and the background portion of the living body 301 shows low luminance (black). Therefore, in the processing for extracting the contour line, the second biometric image 502 is scanned from a lower end thereof as indicated by reference numeral 601, and thus a point at which the luminance value is changed is extracted, thereby detecting contour position coordinates 602. It is noted that although the points at each of which the luminous is firstly changed when the scanning is carried out from the lower end of the second biometric image 502 are extracted as the contour line, alternatively, points (a contour line on the upper side of the finger) at each of which the luminance is changed for the second time may be extracted as the contour line position coordinates.

FIGS. 8A to 8C are respectively views explaining the detection of the inclination of the living body 301 or the bend of the living body 301 from the second biometric image 502. In the case where as shown in FIG. 8A, one approximation straight line 701 is obtained by using the contour position coordinates 602, when the inclination angle of the one approximation straight line 701 does not exceed the prescribed angle, the control portion 110 judges that the living body 301 is disposed straight. On the other hand, when the inclination angle of the one approximation straight line 701 exceeds the prescribed angle, the control portion 110 judges that the living body 301 is disposed to have the inclination. FIG. 8B shows that the living body 301 on the side of the finger root disposing portion 106 is floated with respect to the side of the fingertip disposing portion 105. It is noted that the inclination angle is an angle between the approximation straight line 701 and the lower end of the second biometric image 502.

In addition, when as shown in FIG. 8C, two approximation straight lines 702 and 703 are obtained by using the contour position coordinates 602 of the living body 301, the control portion 110 calculates an angle between the two approximation straight lines 702 and 703. When the resulting angle exceeds a prescribed angle, the control portion 110 judges that the living body 301 is disposed to be bent at the joint. At this time, a position where the two approximation straight lines 702 and 703 intersect with each other is detected as a joint position 704 of the living body 301.

### F. Correction of Biometric Image

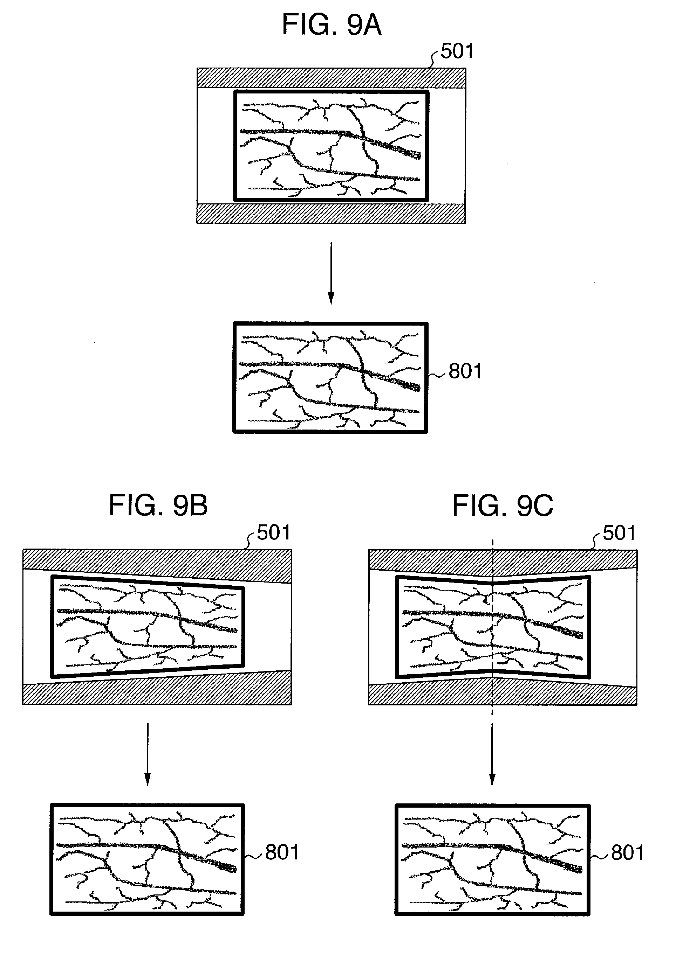

The correction processing for the first biometric image 501 in the inclination correction for the living body 301 in the processing in Step 206 of FIG. 5, and the bend correction for the living body 301 in the processing in Step 207 of FIG. 5 will be described below with reference to FIGS. 8A to 8C, and FIGS. 9A to 9C. When as shown in FIG. 9A, the living body 301 is disposed straight, the state in which the living body 301 is disposed is judged to be suitable. As a result, a biometric image 801 free from the inclination and the bend is directly obtained from the first biometric image 501.

When the living body 301 is disposed to have the inclination, as shown in FIG. 9B, the first biometric image 501 comes to have a trapezoidal shape because the side of the finger root disposing portion 106 of the living body 301 is being floated. Therefore, the correction is carried out in the image processing depending on the inclination angle so that the state of the first biometric image 501 becomes equal to the state in which the living body 301 is disposed straight without being inclined. Carrying out the correction makes it possible to obtain the biometric image free from the inclination. The correction method will now be described in more detail. The inclination angle which is calculated when the approximation straight line shown in FIG. 8B is obtained is used as a technique for correcting the first biometric image 501. Since the inclination angle corresponds to the trapezoidal distortion of the first biometric image 501, a correction coefficient corresponding to the inclination angle is calculated, thereby correcting the trapezoidal distortion.

When the living body 301 is disposed to be bent at the joint, as shown in FIG. 9C, the first biometric image 501 comes to have a shape in which two trapezoids are united with the joint position 704 of the living body 301 as a boundary. Therefore, for the purpose of correcting the image of the first biometric image 501, the first biometric image 501 is divided into right- and left-hand side parts with the joint position 704 of the living body 301 as the boundary, and the resulting right- and left-hand side parts (images) are corrected in the image processing depending on the bend angle so that the state of the living body 301 becomes equal to the state in which the living body 301 is disposed straight, thereby making it possible to obtain the biometric image free from the bend of the living body 301. It is noted that the joint position 704 of the living body 301 is obtained from a point at which the two approximation straight lines 702 and 703 which are calculated during the detection of the bend of the living body 301 intersect with each other. In a word, a point corresponding to the point at which the two approximation straight lines intersect with each other exists on the first biometric image 501, and a straight line passing through this point corresponds to the joint position 704. Inclinations which are calculated when the two approximation straight lines 702 and 703 are calculated are used for the inclination angles corresponding to the correction coefficient when the right- and left-hand side trapezoidal shapes are corrected.

As has been described so far, the detection of the disposition state of the living body 301 is carried out by using the first imaging portion 101 and the second imaging portion 102. When the state of the living body 301 (such as the inclination of the living body 301, or the bend of the living body 301 at the joint) comes to have the shape which is unfavorable for the authentication, it becomes possible to correct the biometric information depending on the state of the living body 301 to obtain the biometric information suitable for the authentication. As a result, it is possible to enhance the convenience of the biometric authentication apparatus.

In addition, the inclination and joint position 704 of the living body 301 can be specified by using the second biometric image 502 which was captured with the second imaging portion 102. Therefore, the correction of the biometric image can be more precisely carried out. As a result, unlike the trapezoid correction which has been conventionally carried out by using a predetermined value, it is possible to carry out the trapezoid correction in which another person acceptance rate is reduced. It is noted that the another person acceptance rate is to accept a living body of another person as the person himself/herself by mistake in the phase of 1 : N authentication, or the like.

### SECOND EMBODIMENT

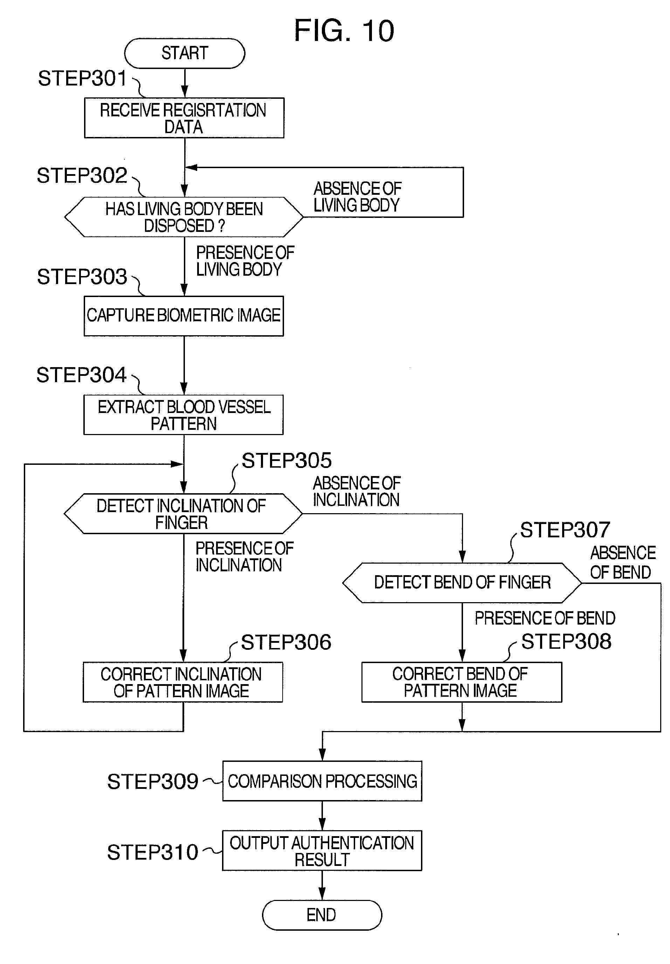

Hereinafter, a second embodiment of the present invention will be described with reference to FIG. 10. In the biometric authentication apparatus 100 according to the first embodiment of the present invention, the extraction of the blood vessel pattern was carried out after the first biometric image 501 had been corrected to obtain the biometric image 801 free from the inclination and the bend in the authentication processing. Contrary to this, however, in the biometric authentication apparatus 100 according to the second embodiment of the present invention, after the blood vessel pattern has been extracted, the correction is carried out for the blood vessel pattern thus extracted, thereby carrying out the comparison processing. It is noted that since a configuration of the biometric authentication apparatus 100 of the second embodiment is substantially the same as that of the biometric authentication apparatus 100 of the first embodiment, a repetitive description thereof is omitted here for the sake of simplicity.

In FIG. 10, in processing in Step 301, the biometric authentication apparatus 100 receives the biometric registration data which has been delivered thereto from the host terminal 200 through the communication 104. At this time, the biometric registration data which has been delivered from the host terminal 200 to the biometric authentication apparatus 100 is the biometric registration data which was previously registered in the registration processing. In processing in Step 302, it is detected that the living body 301 has been disposed on the biometric authentication apparatus 100. While it is judged that the living body 301 has not been disposed on the biometric authentication apparatus 100, the processing in Step 302 is repetitively executed (Step 302: absence of the living body 301). On the other hand, when it is judged that the living body 301 has been disposed on the biometric authentication apparatus 100, the authentication processing proceeds to processing in next Step 303 (Step 302: presence of the living body 301).

In the processing in Step 303, after the quantities of light from the light source portions 103 mounted to the biometric authentication apparatus 100 have been adjusted to the state suitable for the imaging of the living body 301, the biometric images are captured by using the first imaging portion 101 and the second imaging portion 102, respectively. In processing in Step 304, the blood vessel pattern is extracted from the first biometric image 501 to obtain the blood vessel pattern image. In processing in Step 305, the inclination of the living body 301 is detected by using the second biometric image 502 which was captured by imaging the side of the living body 301. When it is detected that the living body 301 is inclined to exceed the prescribed angle, the authentication processing proceeds to processing in Step 306 (Step 305: presence of the inclination). On the other hand, when it is detected that the living body 301 is not inclined to exceed the prescribed angle, the authentication processing proceeds to processing in Step 307 (Step 305: absence of the inclination).

In processing in Step 306, the correction processing for the blood vessel pattern image is executed depending on the inclination of the living body 301. When the living body 301 is disposed on the biometric authentication apparatus 100 to have the inclination, the blood vessel pattern image obtained in the processing in Step 304 becomes an image which is distorted to have the trapezoidal shape depending on the inclination of the living image 301. Therefore, the blood vessel pattern image free from the inclination can be obtained by carrying out the correction in the image processing. After the inclination correction has been carried out for the blood vessel pattern image, the judgment processing in Step 305 is executed again, and then the authentication processing proceeds to processing in next Step 307.

In the processing in Step 307, the detection of the bend of the living body 301 at the joint is carried out by using the second biometric image 502 which was captured by imaging the side of the living body 301. When it is detected that the living body 301 is bent at the joint to exceed the prescribed angle, the authentication processing proceeds to processing in Step 308 (Step 307: presence of the bend). On the other hand, when it is detected that the living body 301 is not bent at the joint to exceed the prescribed angle, the authentication processing proceeds to processing in Step 309 (Step 307: absence of the bend).

In the processing in Step 308, the correction processing for the blood vessel pattern image is executed depending on the bend of the living body 301. When the living body 301 is disposed on the biometric authentication apparatus 100 to be bent, the blood vessel pattern image obtained in the processing in Step 305 becomes the image which is distorted in two directions to have the trapezoidal shapes with the joint position of the living body 301 as a boundary depending on the bend of the living body 301. Therefore, the blood vessel pattern image free from the bend can be obtained by carrying out the correction for the blood vessel pattern image in the image processing.

In processing in Step 309, comparison processing for the blood vessel pattern is executed. The comparison based on the pattern matching is carried out by using the biometric registration data which was previously registered, and the blood vessel pattern image, free from the inclination and the bent, which is obtained until the processing in Step 308, thereby calculating the degree of coincidence.

In processing in Step 310, the judgment for the authentication is carried out based on the degree of coincidence obtained in the processing in Step 309, and the judgment result is outputted to the host terminal 200. The authentication processing in the biometric authentication apparatus 100 of the second embodiment is completed through the ten pieces of processing in Steps 301 to 310.

As has been described so far, the detection of the disposition state of the living body 301 is carried out by using the first imaging portion 101 and the second imaging portion 102. When the state of the living body 301 (such as the inclination of the living body 301, or the bend of the living body 301 at the joint) comes to have the shape which is unfavorable for the authentication, the blood vessel pattern image is corrected depending on the state of the living body 301 to obtain the blood vessel pattern suitable for the authentication. Thereby, it is possible to execute the authentication processing in which the throughput is reduced as compared with the case where the biometric image is corrected. In a word, since the blood vessel pattern image is smaller in size than the first biometric image 501 captured with the first imaging portion 101, the performance (such as the CPU speed) of the control portion 110 can be suppressed as compared with the case where the first biometric image 501 is handled. In addition, since the throughput is reduced, it is possible to shorten the time required for the authentication processing.

### THIRD EMBODIMENT

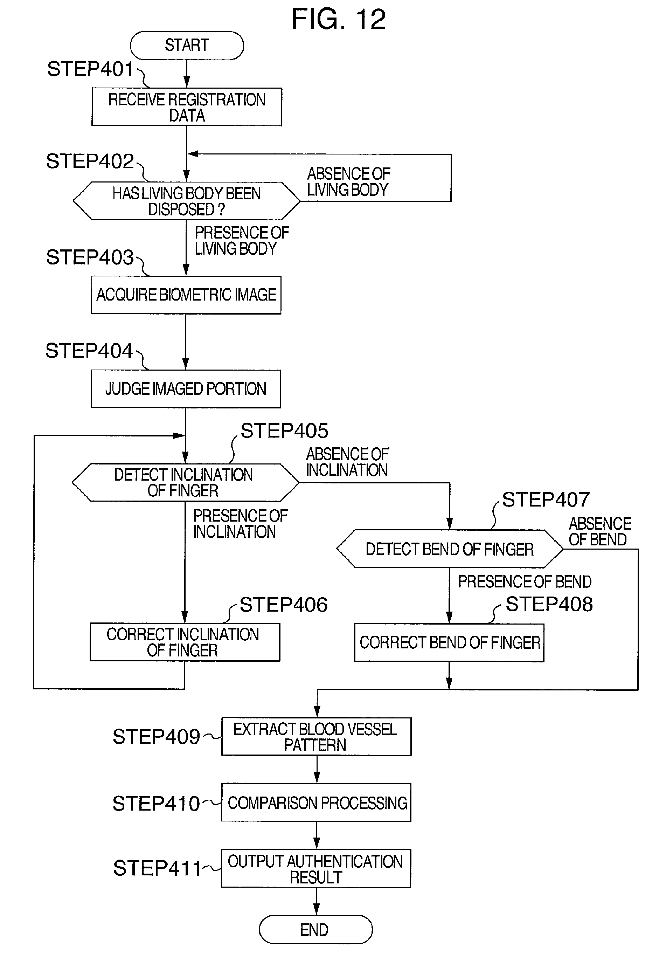

Hereinafter, a third embodiment of the present invention will be described with reference to FIGS. 11 and 12. In the biometric authentication apparatus 100 according to the first embodiment of the present invention, the description was given with respect to the case where as shown in FIG. 6, the first imaging portion 101 captured the first biometric image 501 as the front image of the living body 101, and the second imaging portion 102 captured the second biometric image 502 as the side image of the living body 101. In the biometric authentication apparatus 100 according to the third embodiment of the present invention, the authentication processing in the biometric authentication apparatus 100 will be described below based on the case where as shown in FIG. 11, a living body 1101 is rolled around the axis in the fingertip direction (the axis parallel with a long side of the biometric authentication apparatus 100) from the root of the finger. It is noted that since a configuration of the biometric authentication apparatus 100 of the third embodiment is substantially the same as that of the biometric authentication apparatus 100 of the first embodiment, a repetitive description thereof is omitted here for the sake of simplicity.

FIG. 11 is a view showing a state in which the living body 1101 is rolled clockwise around the axis of the fingertip direction from the root of the finger by a predetermined angle. In this case, unlike the first embodiment of the present invention, the first imaging portion 101 can image the side of the living body 1101, and the second imaging portion 101 can image the front of the living body 1101. In such a manner, when the living body 1101 is rolled, it is necessary to judge which of the images captured with the first imaging portion 101 and the second imaging portion 102, respectively, the front image of the living body 1101 (either the first biometric image or the image for the biometric authentication) is, or which of the images captured with the first imaging portion 101 and the second imaging portion 102, respectively, the side image of the living body 1101 (either the second biometric image or the image for the correction) is.

FIG. 12 is a flow chart explaining an authentication processing flow in the biometric authentication apparatus 100 of the third embodiment. Since three pieces of processing in Steps 401 to 403 in FIG. 12 are the same in description as those of processing in Steps 201 to 203 in FIG. 5 of the first embodiment, a repetitive description thereof is omitted here for the sake of simplicity. After the biometric image has been captured, in order to specify which of the portions of the living body 1101 the images captured with the first imaging portion 101 and the second imaging portion 102, respectively, are, the biometric authentication apparatus 100 carries out the judgment for the imaged portions (Step 404).

A concrete method of judging the imaged portions will now be described. The control portion 110 calculates the approximation straight lines of the contour lines of the living body 1101 with respect to the image captured with the first imaging portion 101, and the image captured with the second imaging portion 102, thereby calculating the inclination angles or bend angles of the living body 1101. Next, the inclination angles or bend angles of the living body 1101 are compared with each other which are respectively calculated from the approximation straight lines of the living body 1101 with respect to the image captured with the first imaging portion 101, and the image captured with the second imaging portion 102. As a result, the image having the larger inclination angle or the larger bend angle is judged to be the image which was captured by imaging the side of the living body 1101. Also, the image having the smaller inclination angle or the smaller bend angle is judged to be the image which was captured by imaging the front of the living body 1101. Since subsequent seven pieces of processing in Steps 405 to 411 are the same in description as those of processing in Steps 204 to 210 of FIG. 5 in the first embodiment, a repetitive description thereof is omitted here for the sake of simplicity.

As has been described so far, the states of the living body 1101 are detected by using the first imaging portion 101 and the second imaging portion 102. Also, the states of the living body 1101 (such as the inclinations of the living body 1101, or the bends of the living body 1101 at the joint) are compared with each other, thereby specifying the portions of the living body 1101 which were imaged by the first imaging portion 101 and the second imaging portion 102, respectively. Thus, it is possible to advance the subsequent pieces of biometric authentication processing. In a word, with the biometric authentication apparatus 100 having the configuration of the third embodiment of the present invention, the processing for the biometric authentication can be executed irrespective of the state of disposition of the living body 1101. As a result, the convenience is enhanced as compared with the related biometric authentication apparatus.

### Modified Change 1

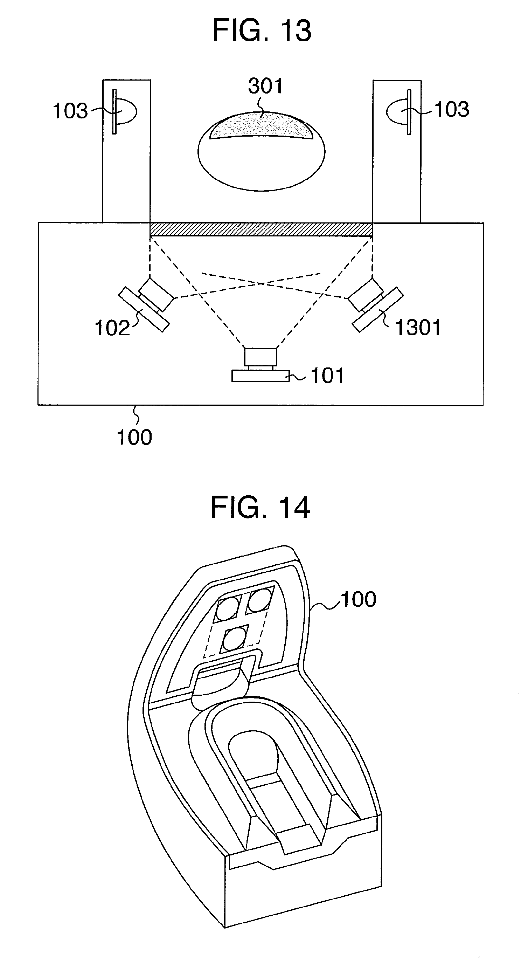

Hereinafter, Modified Change 1 of the first, second and third embodiments will be described with reference to FIG. 13. FIG. 13 is a view showing a structure of a biometric authentication apparatus 100 including a third imaging portion 1301 in addition to the first imaging portion 101 and the second imaging portion 102.

Provision of the third imaging portion 1301 results in that even when the living body 301 is rolled in any direction of the clockwise direction or the counterclockwise direction with respect to the axis passing through the fingertip from the root of the finger, it is possible to advance the authentication processing. In addition, since the number of imaging portions which image the living body 301 is increased to three, an amount of information based on which the inclination angle and the bend angle are calculated is increased, so that it is possible to more precisely correct the biometric image(s).

### Modified Change 2

Next, Modified Change 2 of the embodiments of the present invention will be described with reference to FIG. 14. FIG. 14 is a view showing an external appearance of the biometric authentication apparatus 100 in which the position of the light source portion 103 is changed from the position where the light source portion 103 irradiates the light to the side of the living body 301 to a position where the light source portion 103 irradiates the light to the living body 301 from the obliquely upper portion with respect to the position where the living body 301 is disposed. The light source is disposed obliquely upward with respect to the position where the living body 301 is disposed, which results in that the near infrared light is uniformly irradiated to the living body 301, and thus the tip portion of the living body 301 is prevented from becoming dark. Therefore, it is possible to prevent the reduction of the resolution of the biometric image captured.

### Modified Change 3

Next, Modified Change 3 of the embodiments of the present invention will be described. Modified Change 3 includes two kinds of light source portions 103. That is to say, the light source portions 103 may include an infrared LED which emits the near infrared light in order to acquire the biometric information, and a visible light LED which emits the visible light in order to extract the contour lines based on which the inclination or the bend is judged.

### Modified Change 4

Next, Modified Change 4 of the embodiments of the present invention will be described. Modified Change 4 may include a movable mechanism (not shown). In this case, with this movable mechanism, the first imaging portion 101 may be moved in the phase of capture of the biometric image(s), whereby the first biometric image and the second biometric image can be captured with one imaging portion.

It should be noted that the present invention is by no means limited to the above embodiments or Modified Changes thereof. For example, the embodiments described above have been described in detail in order to describe the present invention clearly, and are not necessarily limited to the embodiments each including all of the constitutions described above. In addition, a part of the constitution of certain embodiment can be replaced with the constitution of any other suitable embodiment, and the constitution of any other suitable embodiment can also be added to the constitution of certain embodiment. Also, the addition, the deletion, and the replacement of any other suitable constitution can be carried out with respect to a part of each of the constitutions of the embodiments described above.

## Claims

1. A biometric authentication apparatus (100) comprising a living body disposing portion (105, 106) on which a living body (301, 1101) of a user is disposed, and a light source portion (103) for irradiating light to the living body (301, 1101) disposed on said living body disposing portion (105, 106), **characterized by** comprising:
an imaging portion (101, 102) for capturing a first biometric image (501) used in biometric authentication, and a second biometric image (502) used in calculation of a correction coefficient used to correct the first biometric image (501); and
a control portion (110) for carrying out control for portions connected thereto, calculating the correction coefficient, and correcting the first biometric image (501) by using the correction coefficient.

2. The biometric authentication apparatus (100) according to claim 1, wherein said control portion (110) extracts one or more approximation straight lines (701, 702, 703) from a contour line of the living body (301, 1101) imaged as the second biometric image (502), calculates an inclination angle of each of the one or more approximation straight lines (701, 702, 703) with an end portion of the second biometric image (502) as a reference, calculates the correction coefficient by using the inclination angle of each of the one or more approximation straight lines (701, 702, 703), and corrects the first biometric image (501).

3. The biometric authentication apparatus (100) according to claim 2, wherein when the first approximation straight line (702) and the second approximation straight line (703) are adapted to be extracted, said control portion (110) obtains a joint position (704) where the first approximation straight line (702) and the second approximation straight line (703) intersect with each other from the second biometric image (502), obtains the correction coefficient based on the inclination angle of the first approximation straight line (702), and the inclination angle of the second approximation straight line (703) with the joint position (704) of the corresponding first biometric image (501) as a boundary, and corrects the first biometric image (501).

4. The biometric authentication apparatus (100) according to claim 2 or 3, wherein said control portion (110) calculates the inclination angle of each of the approximation straight lines (701, 702, 703) from the images captured with said imaging portion (101, 102), and compares the inclination angles of the one or more approximation straight lines (701, 702, 703) with each other, thereby judging which of the first biometric image (501) and the second biometric image (502) each of the images captured with said imaging portion (101, 102) is.

5. The biometric authentication apparatus (100) according to any one of claims 2 to 4, wherein with respect to a feature information image of the living body (301, 1101) created from the first biometric image (501) and the second biometric image (502), said control portion (110) carries out extraction of the inclination angle of each of the one or more approximation straight lines (701, 702, 703), and carries out correction of the image for the biometric authentication.

6. The biometric authentication apparatus (100) according to any one of claims 1 to 5, further comprising:
a communication portion (104) for making communication with a host apparatus (200),
wherein said control portion (110) compares previously registered data for authentication transmitted from said host apparatus (200) through said communication portion (104), and the first biometric image after the correction with each other.

7. The biometric authentication apparatus (100) according to any one of claims 1 to 6, wherein said imaging portion (101, 102, 1301) further captures a third biometric image which is used in calculation of the correction coefficient used to correct the first biometric image (501), and said control portion (110) corrects the first biometric image (501) by using the second biometric image (502) and the third biometric image.

8. A biometric authentication apparatus (100) comprising a living body disposing portion (105, 106) on which a living body (301, 1101) of a user is disposed, **characterized by** comprising:
a light source portion (103) for irradiating light, said light source portion (103) being located on a side of said living body disposing portion (105, 106);
an opening portion (107) through which the light from said light source portion (103) is passed, said opening portion (107) being located below said living body disposing portion (105, 106);
a first imaging portion (101) located below said opening portion (107), and in a position parallel with an optical axis of the light irradiated from said light source portion (103); and
a second imaging portion (102) located near said first imaging portion (101).

9. The biometric authentication apparatus (100) according to claim 8, further comprising:
a control portion (110) for controlling said biometric authentication apparatus (100),
wherein said first imaging portion (101) captures a biometric image on a front side of the living body;
said second imaging portion (102) captures a biometric image on a lateral side of the living body; and
said control portion (110) calculates the correction coefficient used to correct the biometric image of the front side from the biometric image on the lateral side, and corrects the biometric image on the front side by using the correction coefficient.
